# EUROPEAN PATENT APPLICATION

(11) **EP 0 326 526 A1**
(43) Date of publication of application: **02.08.1989**
(21) Application number: 89830023.1
(22) Date of filing: 24.01.1989
(51) Int. Cl.: C07D 401/06, C07D 471/14, A61K 31/55

(54) **Aminoketones of substituted dibenzo- and pyridobenzo-azepinones and pharmaceutical compositions**

(30) Priority: 25.01.1988 IT 1919288
(71) Applicant: ISTITUTO DE ANGELI S.p.A., I-20139 Milano (IT)
(72) Inventor: Gil Quintero, Myrna, I-20122 Milano (IT); Pagani, Ferdinando, I-20050 Verano Brianza Milano (IT); Donetti, Arturo, I-20133 Milano (IT); Schiavi, Giovanni Battista, I-46041 Asola Mantova (IT)
(74) Representative: Aimi, Luciano

(57) **Abstract**

New pharmacologically active aminoketones of substituted dibenzo-and pyridobenzo-azepinones as selective antimuscarinic agents, useful in the treatment of diseases of the gastrointestinal tract of general formula (I) 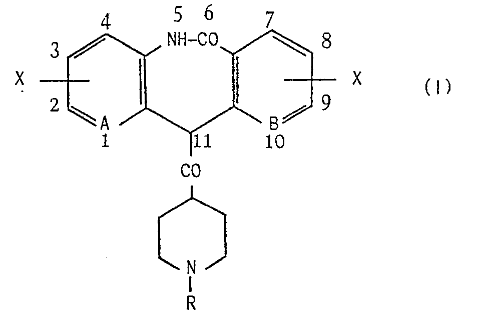 wherein
R represents a C₁₋₄ alkyl,
A and B represent independently nitrogen or carbon with the proviso that A and B cannot be nitrogen at the same time
X represents a substituent at a carbon atom of the fused rings selected from hydrogen, halogen, methyl,
and acid addition salts thereof.

The process for the preparation of the compounds of general formula (I) as well as pharmaceutical compositions containing them are also described.

## Description

The present invention relates to novel pharmacologically active aminoketones of substituted dibenzo- and pyridobenzo-azepinones, to the process for their preparation and to the pharmaceutical compositions containing them. The new compounds are selective antimuscarinic agents useful in the treatment of diseases of gastrointestinal tract.

Antimuscarinic agents may act on peripheral muscurinic receptors (smooth muscle, heart, secretory cells, etc.) and central synapses. Their action is due to interference with the physiological functions of acetylcholine. Atropine has become the prototype of the antimuscarinic agents and has widespread uses as a pharmacological tool. Typical effects produced by atropine are, for example, mydriasis, tachycardia, inhibition of gastric juice and saliva secretion. If atropine is used as a gastric antisecretory agent e.g. in ulcer disease, also unwanted effects of the aforementioned type will occur. Lack of selectivity by atropine is the major drawback that limits the usefulness of this substance for ulcer therapy.

From receptor binding studies pirenzepine has recently demonstrated to be a selective antimuscarinic agent. Competition studies on the binding of tritiated muscarinic antagonists to sub-cellular membrane preparations from a variety of tissues have revealed marked differences between pirenzepine and a number of classical antimuscarine drugs. In particular it has been shown that pirenzepine can distinguish between the receptor subtype governing gastric secretion (neuronal muscarinic receptors: M₁) and those distributed in peripheral tissues (heart, smooth muscle and glands: M₂).

The discovery of pirenzepine has prompted further research in pursuing selective antimuscarine agents with a higher selectivity than the reference pirenzepine molecule.

We have now synthetized, and this is the object of the present invention, a new class of aminoketones of substituted dibenzo- and pyridobenzo-azepinones which have a potent and selective antimuscarinic activity for M₁ muscarinic receptor subtype on the gastrointestinal tract.

The compounds, object of the present invention, have the general formula (I) 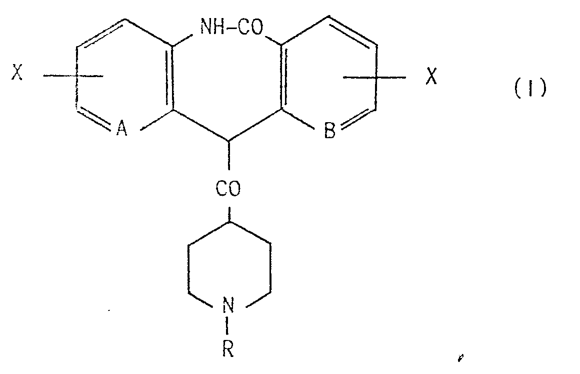 wherein
R represents a C₁₋₄ alkyl,
A and B represent independently nitrogen or carbon with the proviso that A and B cannot be nitrogen at the same time,
X represents a substituent at a carbon atom of the fused rings selected from hydrogen, halogen, methyl.

For pharmaceutical use their physiologically compatible acid addition salts may be also used. The term "acid addition salts" includes salts with organic or inorganic acids. Physiologically compatible acids used for the salification include, for example, hydrochloric, hydrobromic, sulphuric, phosphoric, citric, fumaric, maleic and tartaric acid.

In the present invention the term "halogen" means chlorine, fluorine. Preferred compounds according to the present invention include those wherein R is methyl, X is hydrogen, chlorine and fluorine in position 1, 2, 9 and 10, A and B are independently nitrogen or carbon with the proviso that A and B cannot be nitrogen at the same time and their physiologically compatible acid addition salts. Such compounds generally have better activity than pirenzepina and therefore are preferred as selective antimuscarinic agents and for the treatment of disorders of the gastrointestinal tract.

The compounds of general formula (I) may be prepared by converting "in situ" a compound of general formula (II) 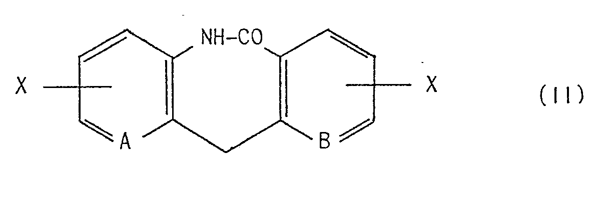 wherein A, B and X are as above defined, into the dilithium salt thereof with organolithium compounds, and then be reacting this salt with a compound of general formula (III) 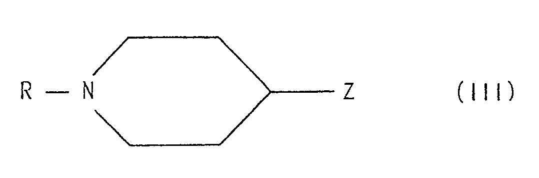 wherein R is as above defined and Z represents a cyano or a CO-Y group in which Y is chlorine, OR₄ or SR₄ in which R₄ is C₁₋₄ alkyl or phenyl. Organolithium compounds used in the conversion "in situ" of the compounds of formula (II) into the dilithium salt include lithium dialkylamides e.g. lithium diisopropylamide or lithium dicyclohexylamide; lithium aryls e.g. phenyl lithium; or alkyllithium, e.g. n-butyllithium alone or in the presence of tetramethyl ethylenediamine. The conversion into the dilithium salt and the subsequent reaction with a compound of formula (III) are performed in an inert organic solvent at temperatures of between -60°C and room temperature, preferably between -20°C and room temperature.

The organic solvents used are those conventionally employed for reactions with organolithium compounds. Particularly advantageous solvents are tetrahydrofuran or others such as diethyl ether, aliphatic hydrocarbons such as hexane, or mixtures of them. If desired cosolvents, such as hexamethyl phosphoric acid triamide, may be also used. After the addition of the organolithium to the compound of general formula (II) has been completed, the compound of formula (III) is added in a stoichiometric amount or a light excess and the reaction mixture is allowed to reach room temperature slowly to complete reaction. Compounds of general formula (I) are isolated from the reaction mixture using conventional procedures. The compounds of general formula (II), used as starting materials in the above described process may be obtained analogously to known methods of preparation, for example, by reacting isocyanates of general formula (IV) 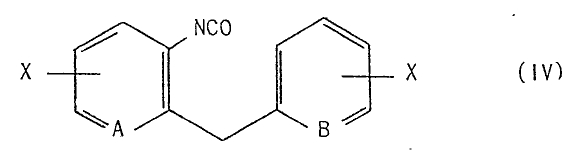 wherein A, B and X are as above defined, with anhydrous aluminium chloride in a suitable solvent such as benzene, toluene, o-dichlorobenzene at temperatures between 100°-180°C.

The compounds of general formula (II), wherein A and B are carbon atoms and X is hydrogen or halogen may also be prepared by reduction of compounds of general formula (V) 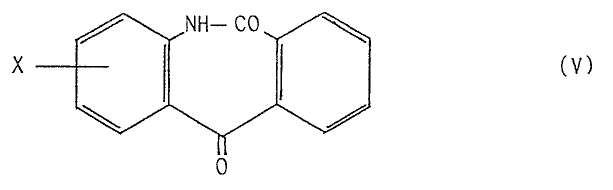 with NaBH₄/CF₃COOH according to the method described by G.W. Coribble et al. in "Synthesis" (1978), 763.

The compounds of general formula (II) wherein A represents nitrogen, B represents carbon and X is hydrogen, may be prepared by catalytic dehalogenation of a compound of formula (VI) 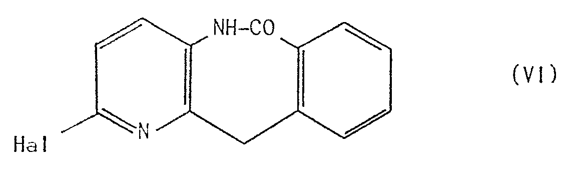 in the presence of a noble metal catalyst e.g. palladium on charcoal in a suitable solvent such as dimethylformamide.

Isocyanates of formula (IV) may be prepared by reacting compounds of general formula (VII) 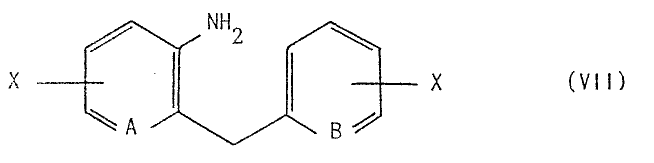 wherein A, B and X are as above defined, with fosgene or difosgene in the presence of an inert solvent e.g. ether, or of an aromatic hydrocarbon e.g. touluene. The reaction may conveniently be effected at a temperature in the range of 40° to 80°.

The compounds of general formula (VII) in turn may be obtained by reduction of compounds of general formula (VIII) 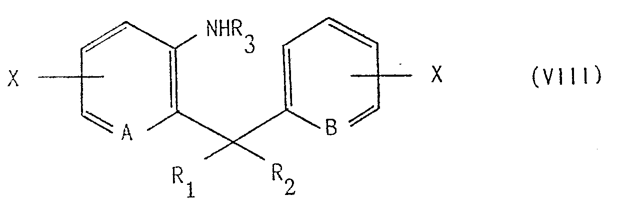 wherein A, B and X are as above defined; R₃ represents an acyl group e.g. t-BuCO, CH₃CO; R₁ and R₂ taken together represent oxygen, or R₁ is hydrogen and R₂ is hydroxyl or vice versa, and by subsequent hydrolysis of the acyl group.

The reduction process of compounds of formula (VIII) may be effected using conventional methods such as catalytic hydrogenation, metal hydride reductions or dissolving metal reductions. The catalytic hydrogenation is usually carried out in the presence of a noble metal catalyst e.g. palladium or platinum on charcoal in alcoholic solvents at room temperature. When a metal hydride reduction is effected, lithium aluminium hydride is preferably used in a suitable solvent such as ether, tetrahydrofurane or dioxane, at a temperature in the range of 30° to 100°C. If a dissolved metal reduction is effected, Zn dust is preferably used in a hot acidic medium such as formic or acetic acid.

If desired, the compounds of formula (VII) wherein A is nitrogen, B is carbon and X represents a halogen atom, may also be obtained by a selective reduction of a compound of formula (IX) 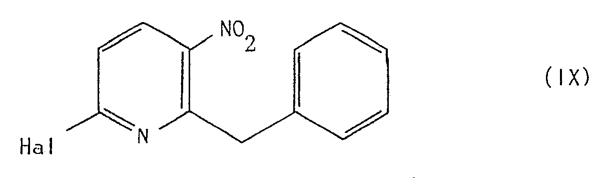 with iron powder in the presence of acetic acid at a temperature in the range of 25° to 50°C.

The compounds of general formula (I), prepared according to the process as above described may optionally be converted with physiologically compatible inorganic or organic acid into the corresponding addition salts, for example, by conventional methods such as by reacting the compounds as bases with a solution of the corresponding acid in a suitable solvent (e.g. ethanol) Particularly preferred acids include for example hydrochloric, fumaric and maleic acid.

Particularly preferred compounds, according to the present invention, are the following:
- 6,11-dihydro-11-[(-methyl-4-piperidinyl)-carbonyl]-5H-dibenz[b,e]-azepin-6-one (Compound 1)
- 1-fluoro-6,11-dihydro-11-[(1-methyl-4-piperidinyl)-carbonyl]-5H-dibenz[b,e]-azepin-6-one (Compound 4)
- 2-chloro-6,11-dihydro-11-[(-methyl-4-piperidinyl)-carbonyl]-5H-pyrido[2,3b]-benzazepin-6-one. (Compound 12)

As already mentioned hereinbefore the new compounds of general formula (I), according to the present invention, have interested pharmacological properties owing to their ability to antagonize the physiological muscarinic effects in warm blooded animals. In particular the compounds have anti-ulcer-antisecretory properties with a good ratio of selectivity between their activity and the undesirable effects (e.g. mydriasis, tachycardia) that commonly occur with therapeutic agents having an antimuscarinic component.

The following tests show that the compounds according to the present invention have favourable characteristics in this respect.

### PHARMACOLOGY

### Antimuscarinic Activity and Selectivity

Antimuscarinic activity and selectivity were examined in vitro by receptor binding studies in two tissues endowed with M₁ and M₂ muscarinic receptors (cerebral cortex, heart) and in vivo by studying the acid secretion and pepsin inhibition (M₁ response) in comparison with the cardiac and mydriatic effects (M₂ response) in conscious dogs equipped with cronic gastric fistula.

### Receptor Binding Studies in Vitro

Muscarinic M₁ activity was determined by studying the displacement of ³H-pirenzepine from cerebral cortex homogenate according to the procedure reported below:
The cerebral cortex donors were male CD-COOBBS rats, 220-250 g body weight. The homogenization process was carried out in a Potter-Evelhjem apparatus in the presence of Na⁺/Mg⁺⁺ HEPES buffer; pH 7.4 (100 mM NaCl, 10 mM MgCl₂, 20 mM HEPES); by filtering the suspension through two layers of cheesecloth. Binding curves for the under study compounds were derived indirectly from competition experiments against 0.5 nM ³H-pirenzepine labelling the muscarinic receptors of the cerebral cortex. 1 ml of the homogenate was incubated for 45 min at 30°C in the presence of a marker ligand and dif ferent concentrations of the cold ligand, conditions under which equilibrium was reached as determined by appropriate association experiments. The incubation was termination by centrifugation (12,000 rpm for 3 min) at room temperature using an Eppendorf microcentrifuge. The resultant pellet was washed twice with 1.5 ml saline to remove the free radioactivity and the final pellet was allowed to drain. The tips of the tubes containing the pellet were cut off and 200µl of tissue subilizer (Lumasolve, Lumac) were added and left to stand overnight. Radioactivity was then counted after addition of 4 ml of liquid scintillation mixture (Dimilume/Toluene 1:10 v:v, Packard).

Assays were carried out in triplicate or quadruplicate and the non-specific binding was defined as the radioactivity bound or entrapped in the pellet when the incubation medium contained 1 µM atropine sulphate. Non-specific binding avaraged less then 30%. K_{D} values (dissociation constants) were obtained by non-linear regression analysis on the basis of one binding site model with TOPFIT-pharmacokinetic programme package (G. HEINZEL "Pharmacokinetics During Drug Development: Data Analysis and Evaluation Techniques" Eds G. BOLZER and J.M. VAN ROSSUM; p. 207, G. Fisher, New York, 1982) after correction for the radioligand occupancy shift according to the equation: K_{D} = IC₅₀/1 + *C/*K_{D}, where *C and *K_{D} represent the concentration and the dissociation constants of the radioligand, used respectively.

Muscarinic M₂ activity was examined by studying the displacement of ³H-NMS from total heart homogenate according to a procedure identical to the one already described hereinbefore for the muscarinic M₁ activity.

### Conscious Gastric Fistula Dog in Vivo

Studies were performed in 5 male Beagle dogs (15-22 Kg) with well established gastric fistula. Gastric secretion was elicited by 2-deoxy-D-glucose (2-DG) (100 mg/kg, infused intravenously in 10 min), and collected by gravity drainage at 15 min intervals. The acid output reached a peak (5.4 mEq/15 min; 75% of the maximal response to histamine) 45 min after the start of 2-DG infusion and then gradually decreased to a plateau level which was about 40% of the peak response (3 hrs after 2-DG). Each sample was measured for volume (to the nearest 0.1 ml) and acidity (titration of 1 ml sample to pH 7 with NaOH 0.1 N by means of an automatic titrator ITT Radiometer PHM 62); the pepsin content in each 15 min sample was determined on 0.1 ml of gastric juice (diluted 1:20 wich HCl 0.1 N), using the hemoglobin digestion method described by Anson (J.Gen. Phys. 22 (1978) 79) and expressed in mU Anson.

The compounds were injected intravenously in a cumulative fashion, each dose being administered at 15 min intervals, starting after the peak response was reached. At each dose level, heart rate was measured by a cardiotachometer and pupil diameter scored under constant light. Doses inhibiting 50% (ED₅₀ and their 95% confidence limits) acid and pepsin output and maximal inhibition (I max), were obtained by fractional linear transformation of the data: D/I = aD+b, were D = dose of antagonist; I = % inhibition of acid or pepsin output; I max = 1/a; ED₅₀ = b/a. (Hirschowitz B.I. Am. J. Physiol. 216: 487-489 1969) Doses increasing heart rate by 50 beats per min (ED₅₀ and their 95% confidence limits) were evaluated by linear regression analysis of the data relating dose to effect.

Doses of compound which at least doubled the initial pupil diameter were also determined.

The results of the testing are set in the following table:

| COMPOUND | IN VITRO RECEPTOR BINDING TEST K_{D} (nM) | | IN VIVO CONSCIOUS GASTRIC FISTULA DOG (µg/kg i.v.) | | | |
|---|---|---|---|---|---|---|
| | M₁ (cortex) | M₂ (heart) | ED₅₀ (agonist 2-DG*) | | Heart rate ED₅₀ (beats/min) | Dose doubling pupil diameter |
| | | | Acid output | Pepsin output | | |
| 1 | 2.1 | 212 | 6.2 | 8.0 | 204 | 512 |
| 4 | 2.2 | 210 | 6.1 | 5.3 | 191 | 512 |
| 12 | 3.15 | 279 | 7.2 | 6.3 | 78 | 320 |

| | | | | | | |
|---|---|---|---|---|---|---|
| * 2-DG = 2-deoxy-D-glucose | | | | | | |

According to a further feature of the present invention there are provided pharmaceutical compositions comprising as active ingredient at least one compound of formula (I), as hereinbefore defined, or a physiologically compatible acid addition salt thereof in association with a pharmaceutical carrier or recipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral, rectal or parenteral administration. Preferred forms include, for example, capsules, tablets, coated tablets, ampoules, suppositories and oral drops.

The active ingredient may be incorporated in excipients or carriers conventionally used in pharmaceutical compositions such as, for example, talc, gum arabic, lactose, gelatine, magnesium stearate, corn starch, acqueous or non-acqueous vehicles, polyvinylpirrolidone, semi-synthetic glicerides of fatty acids, sorbitol, propylen glycol, citric acid, sodium citrate.

The compositions are advantageously formulated at dosage units, each dosage unit being adapted to supply a single dose of the active ingredient. Each dosage unit may conveniently contain from 0.1 mg to 250 mg and preferably from 0.5 mg to 100 mg.

The following examples illustrate some of the new compounds according to the present invention; these examples are not to be in any way considered limitative of the scope of the invention itself:

### Example 1

### 10-fluoro-5H-dibenz[b,e]-azepin-6,11-dihydro-6-one

a) A solution of 2,2-dimethyl-propionanilide (2.8 g; 15.8 mmol) in 30 ml of dry tetrahydrofuran (THF) was cooled in an ice bath under nitrogen. Then a hexane solution of 1.6 M of n-Bu-Li (25 ml; 39.5 mmol) was added dropwise. After stirring for 20 hrs at 25°C, the suspension of the resulting dilithio salt was cooled again in an ice bath and a solution of 2.24 g (23.7 mmol) 2-fluoro-benzaldehyde in 20 ml of dry tetrahydrofuran was added dropwise. The resulting mixture was then stirred at 5°C for 1 hr longer and quenched with a saturated ammonium chloride solution (40ml). The formed layers were separated and the organic phase was dried over anhydrous magnesium sulfate, filtrated and evaporated to dryness under vacuum to give an oil which was purified by flash chromatography to give an oily residue of 2′-(α-hydroxy-2-fluoro-benzyl)-2,2-dimethyl-propionanilide (2 g).
b) A mixture of 2′-(α-hydroxy-2-fluoro-benzyl)-2,2-dimethyl-propionanilide (3 g; 10 mmol), activated zinc dust (1.65 g) in 30 ml of formic acid was refluxed for 12 hrs. The mixture was cooled, filtered and the solvent removed under vacuum giving an oily residue of the crude 2′-(2-fluoro-benzyl)-2,2-dimethyl-propionanilide (2.5 g).
c) A mixture of 2′-(2-fluoro-benzyl)-2,2-dimethyl-propionanilide (2.85 g), acetic acid (40 ml) and concentrated hydrochloric acid (10 ml) was refluxed for 12 hrs. The resulting mixture was cooled, made alkaline with sodium carbonate and extracted with ethyl acetate. The combined organic extracts were dried (MgSO₄) and evaporated to dryness under vacuum giving the crude 2-(2-fluoro-benzyl)-aniline as an oily residue. The oily residue was dissolved in diethyl ether and added to a solution of hydrochloric acid in diethyl ether. From the cooled solution the hydrochloride of 2-(2-fluoro-benzyl)-aniline was isolated as a white solid (2.34 g). M.p. 193°-195°C.
d) To 80 ml of dry dioxane were added 2.6 g of 2-(2-fluoro-benzyl)-aniline hydrochloride and 1.72 ml of trichloromethyl chloroformate. The mixture was heated at 60°C for 24 hrs. The resulting mixture was cooled and evaporated to dryness under vacuum giving the 2-(2-fluoro-benzyl)-phenyl-isocyanate as an oily residue (2.23 g). I R(cm⁻¹): 2270
e) a solution of 2-(2-fluoro-benzyl)-phenyl-isocyanate (1.67 g) in 1,2-dichloro-benzene (30 ml) was added to a mixture of aluminium chloride (4.95 g) in 1,2-dichloro-benzene (60 ml). The resulting mixture was heated at 100°C for 12 hrs. The reaction mixture was cooled, poured into ice/water and extracted with dichloromethane. The organic layer was dried (MgSO₄) and evaporated to dryness under vacuum giving a solid which was crystallized from diethyl ether to give the pure title compound (1.2 g). M.p. 258°-260°C.
   I R (cm⁻¹): 1660 (amide)
   M S (C.I.): 228 m/e [M + H]⁺

The following dibenzazepinones were analogously prepared starting from the appropriate benzaldehyde:
10-chloro-5H-dibenz[b,e]-azepin-6,11-dihydro-6-one
M.p. 267°-268°C.

### Example 2

### 1-chloro-5H-dibenz[b,e]-azepin-6,11-dihydro-6-one

a) a mixture of 2-amino-6-chloro-benzophenones (4.63 g; 20 mmol), activated Zinc dust (3.3 g) in 50 ml of formic acid was refluxed for 12 hrs. The mixture was cooled, filtered and the solvent removed under vacuum giving an oily residue of the crude 2-benzyl-3-chloro-aniline (3.82 g).
b) To a solution of 2-benzyl-3-chloro-aniline (3.5 g) in 50 ml of dry dioxane were added under stirring 2.9 ml of trichloromethyl chloroformate. The mixture was heated at 60°C for 1 hr. The resulting mixture was cooled and evaporated to dryness under vacuum giving the 2-benzyl-3-chloro-phenyl-isocyanate as an oily residue (3.3 g). I R (cm⁻¹): 2270.
c) A solution of 2-benzyl-3-chloro-phenyl-isocyanate (3 g) in 1,2-dichloro-benzene (35 ml) was added to a mixture of aluminium chloride (7.5 g) in 1,2-dichloro-benzene (35 ml). The resulting reaction mixture was refluxed for 4 hrs, cooled and poured into ice/water. The resulting solid was filtered off and dried under vacuum at 50°C to give the pure title compound (2.4 g). M.p. 280°C.
   I R (cm⁻¹): 1660
   M S (C.I.): 244 m/e [M + H]⁺

In a similar way was also prepared:
1-fluoro-5H-dibenz[b,e]-6,11-dihydro-6-one. M.p. 248°C.

### Example 3

### 1-methyl-5H-dibenz[b,e]-azepin-6,11-dihydro-6-one.

a) To a solution of 3-methyl-2-benzyl-aniline (1.62 g) and pyridine (0.7 ml) in 30 ml of dry dioxane was added 1.2 ml of trichloromethylchloroformate. The mixture was heated at 60°C for 2 hrs. The resulting mixture was cooled and filtered from the insoluble material. The mother liquors were concentrated to dryness giving the 3-methyl-2-benzyl-phenyl-isocyanate as an oily residue (1.8 g). I R (cm⁻¹): 2270
b) A solution of 3-methyl-2-benzyl-phenyl-isocyanate (1.8 g) in 10 ml of dry benzene was added to a mixture of aluminium chloride (2.13 g) in dry benzene (15 ml). The resulting mixture was refluxed for 2 hrs. The reaction mixture was then cooled, poured into ice/water and extracted with dichloromethane. The organic layer was washed with water, dried (MgSO₄) and evaporated to dryness under vacuum giving a solid which was crystallized from diethyl ether to give the pure title compound (1.2 g). M.p. 252°-253°C.

The following dibenzazepinones were analogously prepared starting from the appropriate benzyl-aniline:
3-methyl-5H-dibenz[b,e]-azepin-6,11-dihydro-6-one. M.p. 243°C
4-methyl-5H-dibenz[b,e]-azepin-6,11-dihydro-6-one. M.p/ 266°-267°C.

### Example 4

### 1,4-dimethyl-5H-dibenz[b,e]-azepin-6,11-dihydro-6-one.

To a solution magnetically stirred of 1,4-dimethyl-5H-dibenz[b,e]-azepin-6,11-dihydro-6,11-dione (2.5 g) in trifluoroacetic acid (70 ml) at 0°-5°C, under nitrogen were added 2.64 g of sodium borohydride pellets over 30 min. The mixture was stirred under nitrogen at 20°-25°C for 15-30 hrs (to complete the dissolution of the pellets). Additional sodium borohydryde pellets can be added at this point if necessary (T.L.C. analysis). The mixture was diluted with water, cooled in an ice bath, made basic with sodium hydroxide pellets, and extracted with dichloro methane. The organic extract was washed (water, brine), dried (MgSO₄), and concentrated in vacuum to give the title compound (2.35 g). M.p. 270°C.

In a similar way was also prepared:
2-chloro-5H-dibenz[b,e]-azepin-6,11-dihydro-6-one. M.p. 257°C.

### Example 5

### 2-chloro-5H-pyrido[2,3-b]-benzazepin-6,11-dihydro-6-one

a) To a suspension of 2-(α-cyanobenzyl)-3-nitro-6-chloro-pyridine (10 g) in anhydrous ethanol (120 ml), dry HCl was bubbled at room temperature for 2 hrs. Then the resulting solution was concentrated to dryness under vacuum to give a crude solid. The solid was crystallized from hexane to give the pure 2-(α-carbethoxy-benzyl)-3-nitro-6-chloro-pyridine (11.5 g). M.p. 94°-95°C.
b) A solution of 2-(α-carbethoxy-benzyl)-3-nitro-6-chloro-pyridine (10 g) in 200 ml of a mixture of acetic acid/hydrochloric acid (4/1) was refluxed for 2 hrs. The resulting mixture was cooled and evaporated under vacuum to give a solid residue. The residue was taken up with diethyl ether, the insoluble material was discarded and the solvent was concentrated to dryness under vacuum to give a solid of 2-benzyl-3-nitro-6-chloro-pyridine (6.6 g). M.p. 105°C.
c) To a solution of 2-benzyl-3-nitro-6-chloro-pyridine (5 g) in 65 ml of acetic acid were cautiously added 2.5 g of iron (powder) at 25°C. The resulting mixture was stirred at 50°C for 12 hrs. Then the reaction mixture was cooled and concentrated under vacuum. The residue was taken up with water and extracted with diethyl ether. The organic layer was dried (MgSO₄) and evaporated to dryness under vacuum to give a solid of 2-benzyl-3-amino-6-chloro-pyridine (3.74 g). M.p. 117°C.
d) To a solution of 2-benzyl-3-amino-6-chloro-pyridine (3.5 g) in 45 ml of dry dioxane were added under stirring 3 ml of trichloromethylchloroformate. The mixture was heated at 60°C for 1 hr. The resulting mixture was cooled and concentrated to dryness under vacuum to give an oily residue of 2-benzyl-6-chloro-3-pyridin-isocyanate (3.5 g).
   I R (cm⁻¹): 2270.
e) A solution of 2-benzyl-6-chloro-3-pyridin-isocyanate (3 g) in 1,2-dichloro-benzene (45 ml) was added to a mixture of aluminium chloride (5 g) in 1,2-dichloro-benzene (30 ml). The resulting mixture was heated at 50°C for 12 hrs. Then the reaction mixture was cooled, poured into ice/water and extracted with dichloromethane. The organic layer was dried (MgSO₄) and concentrated to dryness under vacuum to give a solid which was cristallized from diethyl ether giving the pure title compound (2.8 g). M.p. 325°C.
   I R (cm⁻¹): 1650
   M S (C.I.): 245 m/e [M + H]⁺

### Example 6

### 6,11-dihydro-11-[(1-methyl-4-piperidinyl)-carbonyl]-5H-dibenz[b,e]-azepin-6-one

### (Compound 1)

To a solution of 5H-dibenz[b,e]-azepin-6,11-dihydro-6-one (5 g; 0.024mol) in 80 ml of dry tetrahydrofuran were added dropwise 34 ml (0.055 mol) of n-butyl-lithium (1.6 M in hexane) with stirring, under nitrogen, at room temperature. The resulting mixture was stirred for 1 hr at 40°C and then 6.16 g (0.036 mol) of ethyl-1-methyl-4-piperidine-carboxylate were added dropwise at -60°C. The mixture was stirred for 30 min longer at -60°C, and then it was allowed to warm to 25°C. The reaction mixture was quenched by addition of an aqueous ammonium chloride solution and the organic layer was separated and concentrated under vacuum. The residue was taken up with water, acidified with diluted hydrochloric acid, and washed with ethyl acetate. The aqueous phase was made alkaline with potassium carbonate. The combined organic extracts were dried (MgSO₄) and evaporated to dryness. The crude product was then purified by flash chromatography on silica gel using a mixture of di chloromethane/methanol as the eluent. From the eluate, 1.5 g of the title compound was obtained as a free base. M.p. 210°C.
I R (cm⁻¹): 3185, 1705, 1650.
M S (C.I.): 335 m/e [M + H]⁺

### Analysis

| | | | | | | | |
|---|---|---|---|---|---|---|---|
| C₂₁H₂₂N₂O₂ | Found % | C | 75.67 | H | 6.62 | N | 8.36 |
| | Calc. % | C | 75.42 | H | 6.63 | N | 8.38 |

NMR δ(CDCl₃): 1.35 - 2.85 (9H, m); 2.10 (3H, s); 4.76 (1H,s); 7.06 - 7.49 (7H, m); 7.98 (1H, m); 9.03 (1H, broad s)

The compound as a base was converted into its hydrochloride by addition of an alcoholic hydrochloric acid solution. M.p. (acetonitrile): 350.1°C. I R (cm⁻¹) : 3180, 1710, 1660
M S (C.I.): 371 m/e [M + H]⁺

### Analysis

| | | | | | | | | | |
|---|---|---|---|---|---|---|---|---|---|
| C₂₁H₂₃ClN₂O₂ | Found % | C | 67.92 | H | 6.23 | N | 7.32 | Cl | 9.45 |
| | Calc. % | C | 68.00 | H | 6.25 | N | 7.55 | Cl | 9.56 |

NMR δ(D₂O): 1.56 - 2.31 (5H, m); 2.79 (3H, s); 2.5 - 3.63 (4H, m); 5.29 (1H, s); 7.09 - 7.84 (8H, m)

According to the above described procedure the following compounds have been obtained starting from the appropriate dibenzazepinones.

### 1-chloro-6,11-dihydro-11-[(1-methyl-4-piperidinyl)-carbonyl]-5H-dibenz [b,e]-azepin-6-one

### (Compound 2)

M.p. 205°C
I R (cm⁻¹) : 3183, 1715, 1660
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.40 | H | 5.78 | N | 7.61 | Cl | 9.68 |
| | Calc. % | C | 68.38 | H | 5.74 | N | 7.60 | Cl | 9.61 |

NMR δ(CDCl₃) : 1.3 - 3 (9H, m); 2.15 (3H, s); 5.63 (1H, s); 7 - 7.66 (6H, m); 8 (1H, m); 10.16 (1H, broad s)

### 2-chloro-6,11-dihydro-11-[(1-methyl-4-piperidinyl)-carbonyl]-5H-dibenz[b,e]-azepin-6-one

### (Compound 3)

M.p. 184°-185°C.
I R (cm⁻¹): 3183, 1715, 1660
M S (C.I.) : 369 m/e [M + H]⁺

### Analysis

| | | | | | | | | | |
|---|---|---|---|---|---|---|---|---|---|
| C₂₁H₂₁ClN₂O₂ | Found % | C | 68.30 | H | 5.72 | N | 7.55 | Cl | 9.70 |
| | Calc. % | C | 68.38 | H | 5.74 | N | 7.60 | Cl | 9.61 |

NMR δ(CDCl₃) : 1 - 3 (9H, m); 2.10 (3H, s); 4.73 (1H, s); 6.8 - 7.7 (6H, m); 7.98 (1H, m); 9.6 (1H, broad s)

### 1-fluoro-6,11-dihydro-11-[(1-methyl-4-piperidinyl)-carbonyl]-5H-dibenz[b,e]-azepin-6-one

### (Compound 4)

M.p. 167°C.
I R (cm⁻¹) 3183, 1720, 1660
M S (C.I.) : 353 m/e [M + H]⁺

### Analysis

| | | | | | | | |
|---|---|---|---|---|---|---|---|
| C₂₁H₂₁FN₂O₂ | Found % | C | 70.78 | H | 5.95 | N | 7.80 |
| | Calc.% | C | 71.57 | H | 6.00 | N | 7.95 |

NMR δ(CDCl₃) : 1.4 - 2.5 (7H, m); 2.12 (3H, s); 2.69 (2H, m); 5.31 (1H, s); 6.7 - 7.6 (6H, m); 7.94 (1H, m); 9.31 (1H, broad s)

### 10-chloro-6,11-dihydro-11-[(1-methyl-4-piperidinyl)-carbonyl]-5H-dibenz[b,e]-azepin-6-one

### (Compound 5)

M.p. 150°C
I R (cm⁻¹) : 3183, 1710, 1655
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 67.68 | H | 5.77 | N | 7.21 | Cl | 9.58 |
| | Calc. % | C | 68.38 | H | 5.74 | N | 7.59 | Cl | 9.61 |

NMR δ(CDCl₃): 1.5 -2.5 (7H, m); 2.19 (3H, s); 2.74 (2H, m); 5.72 (1H, s); 7.0 - 7.6 (5H, m); 7.60 (1H, m); 7.89 (1H, m); 8.64 (1H, broad s)

### 10-fluoro-6,11-dihydro-11-[(1-methyl-4-piperidinyl)-carbonyl]-5H-dibenz[b,e]azepin-6-one

### (Compound 6)

M.p. 223°C
I R (cm⁻¹) : 3183,1720, 1665
M S (C.I.) : 353 m/e [M + H]⁺

### Analysis

| | | | | | | | |
|---|---|---|---|---|---|---|---|
| C₂₁H₂₁FN₂O₂ | Found % | C | 71.10 | H | 6.05 | N | 7.85 |
| | Calc. % | C | 71.57 | H | 6.00 | N | 7.95 |

NMR δ(CDCl₃): 1.4 - 2.5 (7H, m); 2.17 (3H, s); 2.72 (2H, m); 5.37 (1H, s); 7.0 - 7.4 (6H, m); 7.80 (1H, m); 8.78 (1H, s).

### 10-methyl-6,11-dihydro-11-[(1-methyl-4-piperidinyl)-carbonyl]-5H-dibenz[b,e]-azepin-6-one

### (Compound 7)

M.p. : 198°C.
I R (cm⁻¹) : 3183, 1710, 1660
M S (C.I.) : 349 m/e [M + H]⁺

### Analysis

| | | | | | | | |
|---|---|---|---|---|---|---|---|
| C₂₂H₂₄N₂O₂ | Found% | C | 75.43 | H | 6.77 | N | 7.70 |
| | Calc. % | C | 75.83 | H | 6.94 | N | 8.04 |

NMR δ(CDCl₃) : 1.4 - 2.9 (9H, m); 2.18 (3H, s); 2.50 (3H, s); 5.16 (1H,s); 6.9 - 7.4 (6H, m); 7.81 (1H, m); 9.00 (1H, s).

### 1,4-dimethyl-6-11-dihydro-11-[(1-methyl-4-piperidinyl)-carbonyl]-5H-dibenz[b,e]-azepin-6-one

### (Compound 8)

M.p. 222°C
I R (cm⁻¹) : 3183, 1705, 1650
M S (C.I.): 363 m/e [M + H]⁺

### Analysis

| | | | | | | | |
|---|---|---|---|---|---|---|---|
| C₂₃H₂₆N₂O₂ | Found % | C | 75.90 | H | 6.90 | N | 7.43 |
| | Calc. % | C | 76.21 | H | 7.23 | N | 7.73 |

NMR δ(DMSO-d₆ + CDCl₃) : 1.2 - 3.0 (9H, m); 2.12 (3H, s); 2.25 (3H, s); 2.50 (3H, s); 5.19 (1H, s); 6.95 (2H, s); 7.3 - 7.5 (3H, m); 7.72 (1H, d); 9.53 (1H, s)

### 1-methyl-6,11-dihydro-11-[(1-methyl-4-piperidinyl)-carbonyl]- 5H-dibenz[b,e]-azepin-6-one

### (Compound 9)

M.p. 199°C.
I R (cm⁻¹) : 3183, 1710, 1660.
M S (C.I.): 349 m/e [M + H]⁺

### Analysis

| | | | | | | | |
|---|---|---|---|---|---|---|---|
| C₂₂H₂₄N₂O₂ | Found % | C | 75.30 | H | 6.60 | N | 7.75 |
| | Calc. % | C | 75.83 | H | 6.94 | N | 8.04 |

NMR δ(CDCl₃): 1.4 - 3 (9H, m); 2.16 (3H, s); 2.55 (3H, s); 5.07 (1H, s); 6.9 - 7.6 (6H, m); 7.99 (1H, m); 8.69 (1H, s).

### 3-methyl-6,11-dihydro-11-[(1-methyl-4-piperidinyl)-carbonyl]-5H-dibenz[b,e]-azepin-6-one

### (Compound 10)

M.p. 203°C.
I R (cm⁻¹) : 3183, 1710, 1650.
M S (C.I.): 349 m/e [M + H]⁺

### Analysis

| | | | | | | | |
|---|---|---|---|---|---|---|---|
| C₂₂H₂₄N₂O₂ | Found % | C | 75.50 | H | 6.90 | N | 7.79 |
| | Calc. % | C | 75.83 | H | 6.94 | N | 8.04 |

NMR δ(CDCl₃) : 1.3 - 3.0 (9H, m); 2.13 (3H, s); 2.30 (3H, s); 4.77 (1H, s); 6.8 - 7.6 (6H, m); 7.99 (1H, m); 9.60 (1H,s)

### 4-methyl-6,11-dihydro-11-[(1-methyl-4-piperidinyl)-carbonyl]-5H-dibenz[b,e]-azepin-6-one

### (Compound 11)

M.p. 205°C.
I R (cm-1) : 3183, 1710, 1650
M S (C.I.) : 349 m/e [M + H]⁺

### Analysis

| | | | | | | | |
|---|---|---|---|---|---|---|---|
| C₂₂H₂₄N₂O₂ | Found % | C | 75.35 | H | 6.95 | N | 7.75 |
| | Calc. % | C | 75.83 | H | 6.94 | N | 8.04 |

NMR δ(CDCl₃) : 1.4 - 2.9 (9H, m); 2.17 (3H, m); 2.33 (3H, s); 4.76 (1H, s); 7.0 - 7.5 (6H, m); 7.82 (1H, s); 7.96 (1H, m).

### 2-chloro-6,11-dihydro-11-[(1-methyl-4-piperidinyl)-carbonyl]-5H-pyrido[2,3b]-benzazepin-6-one

### (Compound 12)

M.p. 245°C.
I R (cm⁻¹) : 3180, 1710, 1660
M S (C.I.) : 370 m/e [M + H]⁺

### Analysis

| | | | | | | | | | |
|---|---|---|---|---|---|---|---|---|---|
| C₂₀H₂₀ClN₃O₂ | Found % | C | 65.04 | H | 5.74 | N | 11.00 | Cl | 9.35 |
| | Calc. % | C | 64.95 | H | 5.45 | N | 11.36 | Cl | 9.58 |

NMR δ(CDCl₃) : 1.4 - 2.5 (7H, m); 2.14 (3H, s); 2.69 (2H, m); 5.23 (1H, s); 7.1 - 7.6 (3H, m); 7.19 (1H, d); 7.44 (1H, d); 7.94 (1H, m); 9.3 (1H, broad s)

### Example 7

### 2-chloro-6,11-dihydro-11-[(1-methyl-4-piperidinyl)-carbonyl-]5H-pyrido[2,3b]-benzazepin-6-one

### (Compound 12)

To a solution of lithium diisopropylamide (46mmol) [prepared from 6.07 g (0.06 mol) of diisopropilamine and 37.5 ml (0.06 mol) of n-butyl-lithium (1.6 M in hexane) at -10°C] was added dropwise, at 0°C, with stirring, under nitrogen, a solution of 2-chloro-5H-pyrido[2,3b]-benzazepin-6,11-dihydro-6-one ( 5.87 g; 0.024 mol) in 80 ml of dry tetrahydrofuran. The resulting mixture was stirred for one hour at 0°C and then, analogously to Example 6, was made to react with 6.16 g (0.036 mol) of ethyl-1-methyl-4-piperidine-carboxylate. After a work-up similar to that described in the previous example a crude compound was obtained. Pure title compound was obtained by flash chromatography tecnique (eluent: dichloromethane - methanol). Yield 3.3 g. M.p. 245°C.
I R (cm⁻¹) : 3180, 1710, 1660
M S (C.I.) : 370 m/e [M + H]⁺

### Analysis

| | | | | | | | | | |
|---|---|---|---|---|---|---|---|---|---|
| C₂₀H₂₀ClN₃O₂ | Found % | C | 65.22 | H | 5.70 | N | 11.05 | Cl | 9.40 |
| | Calc. % | C | 64.95 | H | 5.45 | N | 11.36 | Cl | 9.58 |

NMR δ(CDCl₃) : 1.4 - 2.5 (7H, m); 2.14 (3H, s); 2.69 (2H, m); 5.23 (1H,s); 7.1 - 7.6 (3H, m); 7.19 (1H, d); 7.44 (1H, d); 7.94 (1H, m); 9.3 (1H, broad s);

According to the above described procedure the following compounds have been obtained:

### 6,11-dihydro-11-[(1-methyl-4-piperidinyl)-carbonyl]-5H-dibenz[b,e]-azepin-6-one

### (Compound 1)

M.p. 210°C
I R (cm⁻¹) : 3185, 1705, 1650
M S (C.I.) : 335 m/e [M + H]⁺

### Analysis

| | | | | | | | |
|---|---|---|---|---|---|---|---|
| C₂₁H₂₂N₂O₂ | Found % | C | 75.32 | H | 6.61 | N | 8.35 |
| | Calc. % | C | 75.42 | H | 6.63 | N | 8.38 |

NMR δ(CDCl₃) : 1.35 - 2.85 (9H, m); 2.10 (3H, s); 4.76 (1H, s); 7.06 - 7.49 (7H, m); 7.98 (1H, m); 9.03 (1H, broad s).

### 1-chloro-6,11-dihydro-11-[(1-methyl-4-piperidinyl)-carbonyl]-5H-dibenz[b,e]-azepin-6-one

### (Compound 2)

M.p. 205°C
I R (cm⁻¹): 3185, 1705, 1650
M S (C.I.) : 369 m/e [M + H]⁺

### Analysis

| | | | | | | | | | |
|---|---|---|---|---|---|---|---|---|---|
| C₂₁H₂₁ClN₂O₂ | Found % | C | 68.42 | H | 5.75 | N | 7.63 | Cl | 9.67 |
| | Calc. % | C | 68.38 | H | 5.74 | N | 7.60 | Cl | 9.61 |

NMR δ(CDCl₃): 1.3 - 3 (9H, m); 2.15 (3H, s); 5.63 (1H, s); 7 - 7.66 (6H,m); 8 (1H, m); 10.16 (1H, broad s)

### 2-chloro-6,11-dihydro-11-[(1-methyl-4-piperidinyl)-carbonyl]-5H-dibenz[b,e]-azepin-6-one

### (Compound 3)

M.p. 184°- 185°C
I R (cm⁻¹): 3183, 1715, 1660
M S (C.I.) : 369 m/e [M + H]⁺

### Analysis

| | | | | | | | | | |
|---|---|---|---|---|---|---|---|---|---|
| C₂₁H₂₁ClN₂O₂ | Found % | C | 68.32 | H | 5.71 | N | 7.56 | Cl | 9.72 |
| | Calc. % | C | 68.38 | H | 5.74 | N | 7.60 | Cl | 9.61 |

NMR δ(CDCl₃): 1 - 3 (9H, m); 2.1 (3H, s); 4.73 (1H, s); 6.8 - 7.7 (6H, m); 7.98 (1H, m); 9.6 (1H, broad s)

### 1-fluoro-6,11-dihydro-11-[(1-methyl-4-piperidinyl)-carbonyl]-5H-dibenz[b,e]-azepin-6-one

### (Compound 4)

M.p. 167°C
I R (cm⁻¹): 3183, 1720, 1660
M S (C.I.) : 353 m/e [M + H]⁺

### Analysis

| | | | | | | | |
|---|---|---|---|---|---|---|---|
| C₂₁H₂₁FN₂O₂ | Found % | C | 71.22 | H | 5.98 | N | 7.78 |
| | Calc. % | C | 71.57 | H | 6.00 | N | 7.95 |

NMR δ(CDCl₃): 1.4 - 2.5 (7H, m); 2.12 (3H, s); 2.69 (2H, m); 5.31 (1H, s); 6.7 - 7.6 (6H, m); 7.94 (1H, m); 9.31 (1H, broad s)

### 10-chloro-6,11-dihydro-11-[(1-methyl-4-piperidinyl)-carbonyl]-5H-dibenz[b,e]-azepin-6-one

### (Compound 5)

M.p. 150°C
I R (cm⁻¹): 3183, 1710, 1655
M S (C.I.) : 369 m/e [M + H]⁺

### Analysis

| | | | | | | | | | |
|---|---|---|---|---|---|---|---|---|---|
| C₂₁H₂₁ClN₂O₂ | Found % | C | 68.40 | H | 5.76 | N | 7.50 | Cl | 9.59 |
| | Calc. % | C | 68.38 | H | 5.74 | N | 7.59 | Cl | 9.61 |

NMR δ(CDCl₃): 1.5 - 2.5 (7H, m); 2.19 (3H, s); 2.74 (2H, m) 5.72 (1H, s); 7.0 - 7.6 (5H, m); 7.60 (1H, m); 7.89 (1H, m); 8.64 (1H, broad s)

### 10-fluoro-6,11-dihydro-11-[(1-methyl-4-piperidinyl)-carbonyl]-5H-dibenz[b,e]-azepin-6-one

### (Compound 6)

M.p. 223°C
I R (cm⁻¹): 3183, 1720, 1665
M S (C.I.) : 353 m/e [M + H]⁺

### Analysis

| | | | | | | | |
|---|---|---|---|---|---|---|---|
| C₂₁H₂₁FN₂O₂ | Found % | C | 71.22 | H | 6.03 | N | 7.86 |
| | Calc. % | C | 71.57 | H | 6.00 | N | 7.95 |

NMR δ(CDCl₃): 1.4 - 2.5 (7H, m); 2.17 (3H, s); 2.72 (2H, m); 5.37 (1H, s); 7.0 - 7.4 (6H, m); 7.80 (1H, m); 8.78 (1H, s)

### 10 methyl-6,11-dihydro-11-[(1-methyl-4-piperidinyl)-carbonyl]-5H-dibenz[b,e]-azepin-6-one

### (Compound 7)

M.p. 198°C
I R (cm⁻¹) : 3183, 1710, 1660
M S (C.I.) : 349 m/e [M + H]⁺

### Analysis

| | | | | | | | |
|---|---|---|---|---|---|---|---|
| C₂₂H₂₄N₂O₂ | Found % | C | 75.52 | H | 6.78 | N | 7.95 |
| | Calc. % | C | 75.83 | H | 6.94 | N | 8.04 |

NMR δ(CDCl₃): 1.4 - 2.9 (9H, m); 2.18 (3H, s); 2.50 (3H, s); 5.16 (1H, s); 6.9 - 7.4 (6H, m); 7.81 (1H, m); 9.00 (1H, s)

### 1,4-dimethyl-6,11-dihydro-11-[(1-methyl-4-piperidinyl)-carbonyl]-5H-dibenz[b,e]-azepin-6-one

### (Compound 8)

M.p. 222°C
I R (cm⁻¹): 3183, 1705, 1650
M S (C.I.) : 363 m/e [M + H]⁺

### Analysis

| | | | | | | | |
|---|---|---|---|---|---|---|---|
| C₂₃H₂₆N₂O₂ | Found % | C | 76.10 | H | 6.98 | N | 7.53 |
| | Calc. % | C | 76.21 | H | 7.23 | N | 7.73 |

NMR δ(DMSO-d₆ + CDCl₃): 1.2 - 3.0 (9H, m); 2.12 (3H, s); 2.25 (3H, s); 2.50 (3H, s); 5.19 (1H, s); 6.95 (2H, s); 7.3 - 7.5 (3H, m); 7.72 (1H, d); 9.53 (1H, s)

### 1-methyl-6,11-dihydro-11-[(1-methyl-4-piperidinyl)-carbonyl]-5H-dibenz[b,e]-azepin-6-one

### (Compound 9)

M.p. 199°C
I R (cm⁻¹): 3183, 1710, 1660
M S (C.I.): 349 m/e [M + H]⁺

### Analysis

| | | | | | | | |
|---|---|---|---|---|---|---|---|
| C₂₂H₂₄N₂O₂ | Found % | C | 74.42 | H | 6.75 | N | 7.92 |
| | Calc. % | C | 75.83 | H | 6.94 | N | 8.04 |

NMR δ(CDCl₃) : 1.4 - 3 (9H, m); 2.16 (3H, s); 2.55 (3H, s); 5.07 (1H, s); 6.9 - 7.6 (6H, m); 7.99 (1H, m); 8.69 (1H, s)

### 3-methyl-6,11-dihydro-11-[(1-methyl-piperidinyl)-carbonyl]-5H-dibenz [b,e]-azepin-6-one

### (Compound 10)

M.p. 203°C
I R (cm⁻¹): 3183, 1710, 1650
M S (C.I.) : 349 m/e [M + H]⁺

### Analysis

| | | | | | | | |
|---|---|---|---|---|---|---|---|
| C₂₂H₂₄N₂O₂ | Found % | C | 74.60 | H | 6.92 | N | 7.80 |
| | Calc. % | C | 75.83 | H | 6.94 | N | 8.04 |

NMR δ(CDCl₃): 1.3 - 3.0 (9H, m); 2.13 (3H, s); 2.30 (3H, s); 4.77 (1H, s); 6.8 - 7.6 (6H, m); 7.99 (1H, m); 9.60 (1H, s)

### 4-methyl-6,11-dihydro-11-[(1-methyl-4-piperidinyl)-carbonyl]-5H-dibenz[b,e]-azepin-6-one

### (Compound 11)

M.p. 205°C
I R (cm⁻¹) : 3183, 1710, 1650
M S (C.I.) : 349 m/e [M + H]⁺

### Analysis

| | | | | | | | |
|---|---|---|---|---|---|---|---|
| C₂₂H₂₄N₂O₂ | Found % | C | 74.45 | H | 6.96 | N | 7.72 |
| | Calc. % | C | 75.83 | H | 6.94 | N | 8.04 |

NMR δ(CDCl₃): 1.4 - 2.9 (9H, m); 2.17 (3H, m); 2.33 (3H, s); 4.76 (1H, s); 7.0 - 7.5 (6H, m); 7.82 (1H, s); 7.96 (1H, m)

### Example 8

### 6,11-dihydro-11-[(1-methyl-4-piperidinyl)-carbonyl]-5H-dibenz[b,e]-azepin-6-one

### (Compound 1)

To a solution of 34 ml (0.055 mol) of n-butyl-lithium (1.6 M in hexane) and 8.3 ml (0.055 mol) of TMEDA (tetramethylethylendiamine) was added dropwise, at 0°C, with stirring, under nitrogen, a solution of 5 g (0.024 mol) of 5H-dibenz[b,e]-azepin-6,11-dihydro-6-one in 80 ml of dry tetrahydrofuran. The resulting mixture was stirred for 1 hr at room temperature and then, analogously to Example 6, was reacted with 6.16 g (0.036 mol) of ethyl-1-methyl-4-piperidine-carboxylate. After a work-up and a purification similar to those already described in the previous examples, the pure title compound was obtained which, according to mixed melting point, TLC, IR, NMR and MS spectrum, was identical to the product obtained and described in Example 6. Yield 2 g.
M.p. 210°C
I R (cm⁻¹): 3185, 1705, 1650
M S (C.I.): 335 m/e [M + H]⁺

### Analysis

| | | | | | | | |
|---|---|---|---|---|---|---|---|
| C₂₁H₂₂N₂O₂ | Found % | C | 75.57 | H | 6.61 | N | 8.35 |
| | Calc. % | C | 75.42 | H | 6.63 | N | 8.38 |

NMR δ(CDCl₃) : 1.35 - 2.85 (9H, m); 2.1 (3H, s); 4.76 (1H, s); 7.06 - 7.49 (7H, m); 7.98 (1H, m); 9.03 (1H, broad s)

Following the above described procedure these analogous compounds were also prepared:

### 1-chloro-6,11-dihydro-11-[(1-methyl-4-piperidinyl)-carbonyl]-5H-dibenz[b,e]-azepin-6-one

### (Compound 2)

M.p. 205°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.41 | H | 5.76 | N | 7.58 | Cl | 9.59 |
| | Calc. % | C | 63.38 | H | 5.74 | N | 7.60 | Cl | 9.61 |

NMR δ(CDCl₃): 1.3 - 3 (9H, m); 2.15 (3H, s); 5.63 (1H, s); 7 - 7.66 (6H, m); 8 (1H, m); 10.16 (1H, broad s)

### 2-chloro-6,11-dihydro-11-[(1-methyl-4-piperidinyl)-carbonyl]-5H-dibenz[b,e]-azepin-6-one

### (Compound 3)

M.p. 184° - 185°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.33 | H | 5.70 | N | 7.57 | Cl | 9.66 |
| | Calc. % | C | 68.38 | H | 5.74 | N | 7.60 | Cl | 9.61 |

NMR δ(CDCl₃): 1 - 3 (9H, m); 2.10 (3H, s); 4.73 (1H, s); 6.8 - 7.7 (6H, m); 7.98 (1H, m); 9.6 (1H, broad s)

### 1-fluoro-6,11-dihydro-11-[(1-methyl-4-piperidinyl)-carbonyl]-5H-dibenz[b,e]-azepin-6-one

### (Compound 4)

M.p. 167°C
I R (cm⁻¹) : 3183, 1705, 1650
M S (C.I.): 353 m/e [M + H]⁺

### Analysis

| | | | | | | | |
|---|---|---|---|---|---|---|---|
| C₂₁H₂₁FN₂O₂ | Found % | C | 70.80 | H | 5.97 | N | 7.82 |
| | Calc. % | C | 71.57 | H | 6.00 | N | 7.95 |

NMR δ(CDCl)₃: 1.4 - 2.5 (7H,m); 2.12 (3H, s); 2.69 (2H, m); 5.31 (1H, s); 6.7 - 7.6 (6H, m); 7.94 (1H, m); 9.31 (1H, broad s)

### 10-chloro-6,11-dihydro-11-[(1-methyl-4-piperidinyl)-carbonyl]-5H-dibenz[b,e]-azepin-6-one

### (Compound 5)

M.p. 150°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 67.98 | H | 5.68 | N | 7.55 | Cl | 9.57 |
| | Calc. % | C | 68.38 | H | 5.74 | N | 7.59 | Cl | 9.61 |

NMR δ(CDCl₃): 1.5 - 2.5 (7H, m); 2.19 (3H, s); 2.74 (2H, m); 5.72 (1H, s); 7.0 - 7.6 (5H, m); 7.60 (1H, m); 7.89 (1H, m); 8.64 (1H, broad s)

### 10-fluoro-6,11-dihydro-11-[(1-methyl-4-piperidinyl)-carbonyl]-5H-dibenz[b,e]-azepin-6-one

### (Compound 6)

M.p. 223°C
I R (cm⁻¹): 3183, 1705, 1650
M S (C.I.) : 353 m/e [M + H]⁺

### Analysis

| | | | | | | | |
|---|---|---|---|---|---|---|---|
| C₂₁H₂₁FN₂O₂ | Found % | C | 71.20 | H | 6.07 | N | 7.88 |
| | Calc. % | C | 71.57 | H | 6.00 | N | 7.95 |

NMR δ(CDCl₃): 1.4 - 2.5 (7H, m); 2.17 (3H, s); 2.72 (2H, m); 5.37 (1H, s); 7.0 - 7.4 (6H, m); 7.80 (1H, m); 8.78 (1H, s)

### 10-methyl-6,11-dihydro-11-[(1-methyl-4-piperidinyl)-carbonyl]-5H-dibenz[b,e]-azepin-6-one

### (Compound 7)

M.p. 198°C
I R (cm⁻¹) : 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.40 | H | 6.81 | N | 7.68 |
| | Calc. % | C | 75.83 | H | 6.94 | N | 8.04 |

NMR δ(CDCl₃): 1.4 - 2.9 (9H, m); 2.18 (3H, s); 2.50 (3H, s); 5.16 (1H, s); 6.9 - 7.4 (6H, m); 7.81 (1H, m); 9.00 (1H, s)

### 1,4-dimethyl-6,11-dihydro-11-[(1-methyl-4-piperidinyl)-carbonyl]-5H-dibenz[b,e]-azepin-6-one

### (Compound 8)

M.p. 222°C
I R (cm⁻¹): 3183, 1705, 1650
M S (C.I.): 363 m/e [M + H]⁺

### Analysis

| | | | | | | | |
|---|---|---|---|---|---|---|---|
| C₂₃H₂₆N₂O₂ | Found % | C | 75.88 | H | 7.18 | N | 7.40 |
| | Calc. % | C | 76.21 | H | 7.23 | N | 7.73 |

NMR δ(DMSO-d₆ + CDCl₃): 1.2 - 3.0 (9H, m); 2.12 (3H, s); 2.25 (3H, s); 2.50 (3H, s); 5.19 (1H, s); 6.95 (2H, s); 7.3 - 7.5 (3H, m); 7.72 (1H, d); 9.53 (1H, s)

### 1-methyl-6,11-dihydro-11-[(1-methyl-4-piperidinyl)-carbonyl]-5H-dibenz[b,e]-azepin-6-one

### (Compound 9)

M.p. 199°C
I R (cm⁻¹): 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.52 | H | 6.62 | N | 7.89 |
| | Calc. % | C | 75.83 | H | 6.94 | N | 8.04 |

NMR δ(CDCl₃): 1.4 - 3 (9H, m); 2.16 (3H, s); 2.55 (3H, s); 5.07 (1H, s); 6.9 - 7.6 (6H, m); 7.99 (1H, m); 8.69 (1H, s)

### 3-methyl-6,11-dihydro-11-[(1-methyl-4-piperidinyl)-carbonyl]-5H-dibenz[b,e]-azepin-6-one

### (Compound 10)

M.p. 203°C
I R (cm⁻¹) : 3183, 1705, 1650
M S (C.I.) : 349 m/e [M + H]⁺

### Analysis

| | | | | | | | |
|---|---|---|---|---|---|---|---|
| C₂₂H₂₄N₂O₂ | Found % | C | 75.45 | H | 6.92 | N | 7.98 |
| | Calc. % | C | 75.83 | H | 6.94 | N | 8.04 |

NMR δ(CDCl₃): 1.3 - 3.0 (9H, m); 2.13 (3H, s); 2.30 (3H, s); 4.77 (1H, s); 6.8 - 7.6 (6H, m); 7.99 (1H, m); 9.60 (1H, s)

### 4-methyl-6,11-dihydro-11-[(1-methyl-4-piperidinyl)-carbonyl]-5H-dibenz[b,e]-azepin-6-one

### (Compound 11)

M.p. 205°C
I R (cm⁻¹): 3183, 1705, 1650
M S (C.I.) : 349 m/e [M + H]⁺

### Analysis

| | | | | | | | |
|---|---|---|---|---|---|---|---|
| C₂₂H₂₄N₂O₂ | Found % | C | 75.32 | H | 6.92 | N | 7.72 |
| | Calc. % | C | 75.83 | H | 6.94 | N | 8.04 |

NMR δ(CDCl₃): 1.4 - 2.9 (9H, m); 2.17 (3H, m); 2.33 (3H, s); 4.76 (1H, s); 7.0 - 7.5 (6H, m); 7.82 (1H, s); 7.96 (1H, m)

### 2-chloro-6,11-dihydro-11-[(1-methyl-4-piperidinyl)-carbonyl]-5H-pyrido[2,3b]-benzazepin-6-one

### (Compound 12)

M.p. 245°C
I R (cm⁻¹): 3180, 1710, 1660
M S (C.I.) : 370 m/e [M + H]⁺

### Analysis

| | | | | | | | | | |
|---|---|---|---|---|---|---|---|---|---|
| C₂₀H₂₀CLN₃O₂ | Found % | C | 65.02 | H | 5.55 | N | 11.12 | Cl | 9.45 |
| | Calc. % | C | 64.95 | H | 5.45 | N | 11.36 | Cl | 9.58 |

NMR δ(CDCl₃): 1.4 - 2.5 (7H, m); 2.14 (3H, s); 2.69 (2H, m); 5.23 (1H, s); 7.1 - 7.6 (3H, m); 7.19 (1H, d); 7.44 (1H, d); 7.94 (1H, m); 9.3 (1H, broad s)

### Example 9

### 1-chloro-6,11-dihydro-11-[(1-methyl-4-piperidinyl)-carbonyl]-5H-dibenz[b,e]-azepin-6-one

### (Compound 2)

To a solution of 1-chloro-5H-dibenz[b,e]-azepin-6,11-dihydro-6-one (5.84 g, 0.024 mol) in 80 ml of dry tetrahydrofuran were added dropwise 27.5 ml (0.055 mol) of phenyl lithium [2 M in benzene/ether (7/3)] with stirring, under nitrogen at room temperature. The mixture was stirred for 1 hr at 40°C and then analogously to Example 6, 6.16 g (0.036 mol) of ethyl-1-methyl-4-piperidine-carboxylate were added. After a similar work-up and purification (flash chromatography) to those already described in previous examples, the pure title compound was obtained. Yield: 1.75 g. M.p. 205°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.36 | H | 5.75 | N | 7.63 | Cl | 9.65 |
| | Calc. % | C | 68.38 | H | 5.74 | N | 7.60 | Cl | 9.61 |

NMR δ(CDCl₃): 1.3-3 (9H, m); 2.15 (3H, s); 5.63 (1H, s); 7 - 7.66 (6H, m); 8 (1H, m); 10.16 (1H, broad s)

According to the above described procedure the following compounds have been obtained:

### 6,11-dihydro-11-[(1methyl-4-piperidinyl)-carbonyl]-5H-dibenz[b,e]-azepin-6-one

### (Compound 1)

M.p. 210°C
I R (cm⁻¹) : 3185, 1705, 1650
M S (C.I.): 335 m/e [M + H]⁺

### Analysis

| | | | | | | | |
|---|---|---|---|---|---|---|---|
| C₂₁H₂₂N₂O₂ | Found % | C | 75.38 | H | 6.60 | N | 8.35 |
| | Calc. % | C | 75.42 | H | 6.63 | N | 8.38 |

NMR δ(CDCl₃): 1.35 - 2.85 (9H, m); 2.10 (3H, s); 4.76 (1H, s); 7.06 - 7.49 (7H, m); 7.98 (1H, m); 9.03 (1H, broad s)

### 2-chloro-6,11-dihydro-11-[(1-methyl-4-piperidinyl)-carbonyl]-5H-dibenz[b,e]-azepin-6-one

### (Compound 3)

M.p. 184° - 185°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.29 | H | 5.71 | N | 7.45 | Cl | 9.59 |
| | Calc. % | C | 68.38 | H | 5.74 | N | 7.60 | Cl | 9.61 |

NMR δ(CDCl₃): 1 - 3 (9H, m); 2.10 (3H, s); 4.73 (1H, s); 6.8 - 7.7 (6H, m); 7.98 (1H, m); 9.6 (1H, broad s)

### 1-fluoro-6,11-dihydro-11-[(1-methyl-4-piperidinyl)-carbonyl]-5H-dibenz[b,e]-azepin-6-one

### (Compound 4)

M.p. 167°C
I R (cm⁻¹): 3183, 1705, 1650
M S (C.I.): 353 m/e [M + H]⁺

### Analysis

| | | | | | | | |
|---|---|---|---|---|---|---|---|
| C₂₁H₂₁FN₂O₂ | Found % | C | 70.32 | H | 5.88 | N | 7.89 |
| | Calc. % | C | 71.57 | H | 6.00 | N | 7.95 |

NMR δ(CDCl₃): 1.4 - 2.5 (7H, m); 2.12 (3H, s); 2.69 (2H, m); 5.31 (1H, s); 6.7 - 7.6 (6H, m); 7.94 (1H, m); 9.31 (1H, broad s)

### 10-chloro-6,11-dihydro-11-[(1-methyl-4-piperidinyl)-carbonyl]-5H-dibenz[b,e]-azepin-6-one

### (Compound 5)

M.p. 150° C
I R (cm⁻¹) : 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 67.88 | H | 5.76 | N | 7.22 | Cl | 9.55 |
| | Calc. % | C | 68.38 | H | 5.74 | N | 7.59 | Cl | 9.61 |

NMR δ(CDCl₃) : 1.5 - 2.5 (7H, m); 2.19 (3H, s); 2.74 (2H, m); 5.72 (1H, s); 7.0 - 7.6 (5H, m); 7.60 (1H, m); 7.89 (1H, m); 8.64 (1H, broad s)

### 10-fluoro-6,11-dihydro-11-[(1-methyl-4-piperidinyl)-carbonyl]-5H-dibenz[b,e]-azepin-6-one

### (Compound 6)

M.p. 223°
I R (cm⁻¹) : 3185, 1705, 1650
M S (C.I.): 353 m/e [M + H]⁺

### Analysis

| | | | | | | | |
|---|---|---|---|---|---|---|---|
| C₂₁H₂₁ClN₂O₂ | Found % | C | 71.22 | H | 5.96 | N | 7.78 |
| | Calc. % | C | 71.57 | H | 6.00 | N | 7.95 |

NMR δ(CDCl₃): 1.4 - 2.5 (7H , m); 2.17 (3H, s); 2.72 (2H, m); 5.37 (1H, s); 7.0 - 7.4 (6H, m); 7.80 (1H, m); 8.78 (1H, s)

### 10-methyl-6,11-dihydro-11-[(1-methyl-4-piperidinyl)-carbonyl]-5H-dibenz[b,e]-azepin-6-one

### (Compound 7)

M.p. 198°C
I R (cm⁻¹): 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.55 | H | 6.82 | N | 7.85 |
| | Calc. % | C | 75.83 | H | 6.94 | N | 8.04 |

NMR δ(CDCl₃): 1.4 - 2.9 (9H, m); 2.18 (3H, s); 2.50 (3H, s); 5.16 (1H, s); 6.9 - 7.4 (6H, m); 7.81 (1H, m); 9.00 (1H, s)

### 1,4-dimethyl-6,11-dihydro-11-[(1-methyl-4-piperidinyl)-carbonyl]-5H-dibenz[b,e]-azepin-6-one

### (Compound 8)

M.p. 222°C
I R (cm⁻¹): 3183, 1705, 1650
M S (C.I.) : 363 m/e [M + H]⁺

### Analysis

| | | | | | | | |
|---|---|---|---|---|---|---|---|
| C₂₃H₂₆N₂O₂ | Found % | C | 75.92 | H | 7.26 | N | 7.51 |
| | Calc. % | C | 76.21 | H | 7.23 | N | 7.73 |

NMR δ(DMSO-d₆ + CDCl₃): 1.2 - 3.0 (9H, m); 2.12 (3H, s); 2.25 (3H, s); 2.50 (3H, s); 5.19 (1H, s); 6.95 (2H, s); 7.3 - 7.5 (3H, m); 7.72 (1H, d); 9.53 (1H, s)

### 1-methyl-6,11-dihydro-11-[(1-methyl-4-piperidinyl)-carbonyl]-5H-dibenz[b,e]-azepin-6-one

### (Compound 9)

M.p. 199°C
I R (cm⁻¹) : 3183, 1705, 1650
M S (C.I.): 349 m/e [M + E]⁺

### Analysis

| | | | | | | | |
|---|---|---|---|---|---|---|---|
| C₂₂H₂₄N₂O₂ | Found % | C | 75.52 | H | 6.96 | N | 7.72 |
| | Calc. % | C | 75.83 | H | 6.94 | N | 8.04 |

NMR δ(CDCl₃) : 1.4 - 3 (9H, m); 2.16 (3H, s); 2.55 (3H, s); 5.07 (1H, s); 6.9 - 7.6 (6H, m); 7.99 (1H, m); 8.69 (1H, s)

### 3-methyl-6,11-dihydro-11-[(1-methyl-4-piperidinyl)-carbonyl]-5H-dibenz[b,e]-azepin-6-one

### (Compound 10)

M.p. 203°C
I R (cm⁻¹) : 3183, 1705, 1650
M S (C.I.) : 349 m/e [M + H]⁺

### Analysis

| | | | | | | | |
|---|---|---|---|---|---|---|---|
| C₂₂H₂₄N₂O₂ | Found % | C | 75.48 | H | 6.93 | N | 7.95 |
| | Calc. % | C | 75.83 | H | 6.94 | N | 8.04 |

NMR δ(CDCl₃) : 1.3 - 3.0 (9H, m); 2.13 (3H, s); 2.30 (3H, s); 4.77 (1H, s); 6.8 - 7.6 (6H, m); 7.99 (1H, m); 9.60 (1H, s)

### 4-methyl-6,11-dihydro-11-[(1-methyl-4-piperidinyl)-carbonyl]-5H-dibenz[b,e]-azepin-6-one

### (Compound 11)

M.p. 205°C
I R (cm⁻¹): 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.48 | H | 6.88 | N | 8.06 |
| | Calc. % | C | 75.83 | H | 6.94 | N | 8.04 |

NMR δ(CDCl₃) : 1.4 - 2.9 (9H, m); 2.17 (3H, m); 2.33 (3H, s); 4.76 (1H, s); 7.0 - 7.5 (6H, m); 7.82 (1H, s); 7.96 (1H, m)

### 2-chloro-6,11-dihydro-11-[(1-methyl-4-piperidinyl)-carbonyl]-5H-pyrido[b,e]-benzeazepin-6-one

### (Compound 12)

M.p. 245°C
I R (cm⁻¹): 3180, 1710, 1660
M S (C.I.): 370 m/e [M + H]⁺

### Analysis

| | | | | | | | | | |
|---|---|---|---|---|---|---|---|---|---|
| C₂₀H₂₀ClN₃O₃ | Found % | C | 64.88 | H | 5.55 | N | 11.12 | Cl | 9.33 |
| | Calc. % | C | 64.95 | H | 5.45 | N | 11.36 | Cl | 9.58 |

NMR δ(CDCl₃): 1.4 - 2.5 (7H, m); 2.14 (3H, s); 2.69 (2H, m); 5.23 (1H, s); 7.1 - 7.6 (3H, m); 7.19 (1H, d); 7.44 (1H, d: 7.94 (1H, m); 9.3 (1H, broad s)

### Example 10

### 6,11-dihydro-11-[(1-methyl-4-piperidinyl)-carbonyl]-5H-dibenz[b,e]-azepin-6-one

### (Compound 1)

To a solution of 5H-dibenz[b,e]-azepin-6,11-dihydro-6-one (5 g; 0.024 mol) in 80 ml of dry tetrahydrofuran were added dropwise 34 ml (0.055 mol) of n-butyl-lithium (1.6 M in hexane) with stirring, under nitrogen, at room temperature. The resulting mixture was stirred for one hour at 40°C, and then 3.58 g (0.029 mol) of 1-methyl-4-cyano-piperidine were added dropwise to the solution kept at -60°C. The mixture was stirred for further 30 min at -60°C and then allowed to warm to room temperature. The reaction mixture was quenched by addition of a solution of hydrochloric acid and stirred for one one at room temperature. the organic layer was separated and concentrated under vacuum. The residue was taken up with water, and washed with ethyl acetate. The aqueous phase was made alkaline with potassium carbonate and extracted with dichloromethane. The combined organic extracts were dried (MgSO₄) and evaporated to dryness. The crude product was purified by flash chromatography (eluent: dichloromethane - methanol) to give the pure title compound, which according to mixed melting point, TCL, IR, NMR, MS spectrum was identical to the product obtained and described in Example 6. Yield: 1 g. M.p. 210°C
I R (cm-1): 3185, 1705, 1650
M S (C.I.): 335 m/e [M + H]⁺

### Analysis

| | | | | | | | |
|---|---|---|---|---|---|---|---|
| C₂₁H₂₂N₂O₂ | Found % | C | 75.38 | H | 6.60 | N | 8.35 |
| | Calc. % | C | 75.42 | H | 6.63 | N | 8.38 |

NMR δ(CDCl₃) : 1.35 - 2.85 (9H, m); 2.10 (3H, s);4.76 (1H, s); 7.06 - 7.49 (7H, m); 7.98 (1H, m); 9.03 (1H, broad s)

Following the above described procedure, these analogous compounds were also prepared :

### 1-chloro-6,11-dihydro-11-[(1-methyl-4-piperidinyl)-carbonyl]-5H-dibenz[b,e]-azepin-6-one

### (Compound 2)

M.p. 205°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.27 | H | 5.70 | N | 7.70 | Cl | 9.58 |
| | Calc. % | C | 68.38 | H | 5.74 | N | 7.60 | Cl | 9.61 |

NMR δ(CDCl₃): 1.3 - 3 (9H,m); 2.15 (3H, s); 5.63 (1H, s); 7 - 7.66 (6H, m); 8 (1H, m); 10.16 (1H, broad s)

### 2-chloro-6,11-dihydro-11-[(1-methyl-4-piperidinyl)-carbonyl]-5H-dibenz[b,e]-azepin-6-one

### (Compound 3)

M.p. 184°C - 185°C
I R (cm⁻¹): 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.27 | H | 5.73 | N | 7.57 | Cl | 9.68 |
| | Calc. % | C | 68.38 | H | 5.74 | N | 7.60 | Cl | 9.61 |

NMR δ(CDCl₃): 1 - 3 (9H, m); 2.10 (3H, s); 4.73 (1H, s); 6.8 - 7.7 (6H, m); 7.98 (1H, m); 9.6 (1H broad s)

### 1-fluoro-6,11-dihydro-11-[(1-methyl-4-piperidinyl)-carbonyl]-5H-dibenz[b,e]-azepin-6-one

### (Compound 4)

M.p. 167° C
I R (cm⁻¹): 3183, 1705, 1650
M S (C.I.) : 353 m/e [M + H]⁺

### Analysis

| | | | | | | | |
|---|---|---|---|---|---|---|---|
| C₂₁H₂₁FN₂O₂ | Found % | C | 71.22 | H | 5.92 | N | 7.83 |
| | Calc. % | C | 71.57 | H | 6.00 | N | 7.95 |

NMR δ(CDCl₃): 1.4 - 2.5 (7H, m); 2.12 (3H, s); 2.69 (2H, m); 5.31 (1H, s); 6.7 - 7.6 (6H, m); 7.94 (1H, m); 9.31 (1H, broad s)

### 10-chloro-6,11-dihydro-11-[(1-methyl-4-piperidinyl)-carbonyl]-5H-dibenz[b,e]-azepin-6-one

### (Compound 5)

M.p. 150°C
I R (cm⁻¹) : 3183, 1705, 1650
M S (C.I.): 369 m/e [M + H]⁺

### Analysis

| | | | | | | | | | |
|---|---|---|---|---|---|---|---|---|---|
| C₂₁H₂₁ClN₂O₂ | Found % | C | 68.12 | H | 5.76 | N | 7.48 | Cl | 9.50 |
| | Calc. % | C | 68.38 | H | 5.74 | N | 7.59 | Cl | 9.61 |

NMR δ(CDCl₃): 1.5 - 2.5 (7H, m); 2.19 (3H, s); 2.74 (2H, m); 5.72 (1H, s); 7.0 - 7.6 (5H, m); 7.60 (1H, m); 7.89 (1H, m); 8.64 (1H, broad s)

### 10-fluoro-6,11-dihydro-11-[(1-methyl-4-piperidinyl)-carbonyl]-5H-dibenz[b,e]-azepin-6-one

### (Compound 6)

M.p. 223°C
I R (cm⁻¹) : 3183, 1705, 1650
M S (C.I.) : 353 m/e [M + H]⁺

### Analysis

| | | | | | | | |
|---|---|---|---|---|---|---|---|
| C₂₁H₂₁FN₂O₂ | Found % | C | 71.36 | H | 5.87 | N | 8.02 |
| | Calc. % | C | 71.57 | H | 6.00 | N | 7.95 |

NMR δ(CDCl₃): 1.4 - 2.5 (7H, m); 2.17 (3H, s); 2.72 (2H, m); 5.37 (1H, s); 7.0 - 7.4 (6H, m); 7.80 (1H, m); 8.78 (1H, s)

### 10-methyl-6,11-dihydro-11-[(1-methyl-4-piperidinyl)-carbonyl]-5H-dibenz[b,e]-azepin-6-one

### (Compound 7)

M.p. 198°C
I R (cm⁻¹): 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.45 | H | 6.82 | N | 7.87 |
| | Calc. % | C | 75.83 | H | 6.94 | N | 8.04 |

NMR δ(CDCl₃): 1.4 - 2.9 (9H, m); 2.18 (3H, s); 2.50 (3H, s); 5.16 (1H, s); 6.9 - 7.4 (6H, m); 7.81 (1H, m); 9.00 (1H, s)

### 1,4-dimethyl-6,11-dihydro-11-[(1-methyl-4-piperidinyl)-carbonyl]-5H-dibenz[b,e]-azepin-6-one

### (Compound 8)

M.p. 222°C
I R (cm⁻¹): 3183, 1705, 1650
M S (C.I.): 363 m/e [M + H]⁺

### Analysis

| | | | | | | | |
|---|---|---|---|---|---|---|---|
| C₂₃H₂₆N₂O₂ | Found % | C | 75.92 | H | 7.30 | N | 7.45 |
| | Calc. % | C | 76.21 | H | 7.23 | N | 7.73 |

NMR δ(DMCO-d₆ + CDCl₃): 1.2 - 3.0 (9H, m); 2.12 (3H, s); 2.25 (3H, s); 2.50 (3H, s); 5.19 (1H, s); 6.95 (2H, s); 7.3 - 7.5 (3H, m); 7.72 (1H, d); 9.53 (1H, s)

### 1-methyl-6,11-dihydro-11-[(1-methyl-4-piperidinyl)-carbonyl]-5H-dibenz[b,e]-azepin-6-one

### (Compound 9)

M.p. 199°C
I R (cm⁻¹): 3183,1705, 1650
M S (C.I.) : 349 m/e [M + H]⁺

### Analysis

| | | | | | | | |
|---|---|---|---|---|---|---|---|
| C₂₂H₂₄N₂O₂ | Found % | C | 75.65 | H | 6.88 | N | 8.02 |
| | Calc. % | C | 75.83 | H | 6.94 | N | 8.04 |

NMR δ(CDCl₃): 1.4 - 3 (9H, m); 2.16 (3H, s); 2.55 (3H, s); 5.07 (1H, s); 6.9 - 7.6 (6H, m); 7.99 (1H, m); 8.69 (1H, s)

### 3-methyl-6,11-dihydro-11-[(1-methyl-4-piperidinyl)-carbonyl]-5H-dibenz[b,e]-azepin-6-one

### (Compound 10)

M.p. 203°C
I R (cm⁻¹) : 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.62 | H | 6.88 | N | 7.98 |
| | Calc. % | C | 75.83 | H | 6.94 | N | 8.04 |

NMR δ(CDCl₃): 1.3 - 3.0 (9H, m); 2.13 (3H, s); 2.30 (3H, s); 4.77 (1H, s); 6.8 - 7.6 (6H, m); 7.99 ( 1H, m); 9.60 (1H, s)

### 4-methyl-6,11-dihydro-11-[(1-methyl-4-piperidinyl)-carbonyl]-5H-dibenz[b,e]-azepin-6-one

### (Compound 11)

M.p. 205°C
I R (cm⁻¹): 3183, 1705, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.62 | H | 6.92 | N | 7.98 |
| | Calc. % | C | 75.83 | H | 6.94 | N | 8.04 |

NMR δ(CDCl₃): 1.4 - 2.9 (9H,m); 2.17 (3H, m); 2.33 (3H, s); 4.76 (1H, s); 7.0 - 7.5 (6H, m); 7.82 (1H, s); 7.96 (1H, m)

### 2-chloro-6,11-dihydro-11-[(1-methyl-4-piperidinyl)-carbonyl]-5H-pyrido[2,3b]-benzazepin-6-one

### (Compound 12)

M.p. 245°C
I R (cm⁻¹): 3180, 1710, 1660
M S (C.I.): 370 m/e [M + H]⁺

### Analysis

| | | | | | | | | | |
|---|---|---|---|---|---|---|---|---|---|
| C₂₀H₂₀ClN₃O₃ | Found % | C | 64.75 | H | 5.38 | N | 11.27 | Cl | 9.48 |
| | Calc. % | C | 64.95 | H | 5.45 | N | 11.36 | Cl | 9.58 |

NMR δ(CDCl₃): 1.4 - 2.5 (7H, m); 2.14 (3H, s); 2.69 (2H, m); 5.23 (1H, s); 7.1 - 7.6 (3H, m); 7.19 (1H, d); 7.44 (1H, d); 7.94 (1H, m); 9.3 (1H, broad s)

### Example 11

### 6,11-dihydro-11-[(1-methyl-4-piperidinyl)-carbonyl]-5H-pyrido[2,3b]-benzazepin-6-one (Compound 13)

A solution of 2-chloro-6,11-dihydro-11-[(1-methyl-4-piperidinyl)-carbonyl]-5H-pyrido[2,3b]-benzazepin-6-one (1 g; 0.0027 mol) and 1.32 mol of TEA (Triethylamine) in 100 ml of dry dimethylformamide was hydrogenated at room temperature and atmospheric pressure in the presence of 10% Pd/C. The catalyst was then filtered off and the solvent removed under vacuum. The title compound was obtained after crystallization from diethyl ether/hexane. Yield: 0.4 g. M.p. 188° - 190°C.
I R (cm⁻¹) : 3180, 1710, 1660
M S (C.I.): 336 m/e [M + H]⁺

### Analysis

| | | | | | | | |
|---|---|---|---|---|---|---|---|
| C₂₀H₂₁N₃O₂ | Found % | C | 71.22 | H | 6.08 | N | 12.10 |
| | Calc. % | C | 71.62 | H | 6.31 | N | 12.52 |

NMR δ(DMSO-d₆ + CDCl₃): 1.2 - 2.4 (7H,m); 2.09 (3H ,s); 2.70 (2H, m); 5.42 (1H, s); 7.1 - 7.5 (4H, m); 7.21 (1H, m); 7.82 (1H, d); 8.30 (1H, m); 10.32 (1H, s)

### Example 12

### 6,11-dihydro-11-[(1-ethyl-4-piperidinyl)-carbonyl]-5H-dibenz[b,e]-azepin-6-one

### (Compound 14)

To a solution of 5H-dibenz[b,e]-azepin-6,11-dihydro-6-one (5 g; 0.0024 mol) in 80 mol of dry tetrahydrofuran were added dropwise 34 ml (0.055 mol) of n-Butyl-lithium (1.6 M in hexane) with stirring, under nitrogen, at room temperature. The resulting mixture was stirred for one hour at 40°C and then 6.16 g (0.036 mol) of methyl-1-ethyl-4-piperidine-carboxylate were added dropwise at -60°C, and then it was allowed to warm to 25°C. The reaction mixture was quenched with an aqueous ammonium chloride solution. The following work-up was similar to the one already described in Example 6. The crude product was purified by flash chromatography (eluent: dichloromethane -methanol) to give the pure title compound. Yield: 1.4 g. M.p. 242°C.
I R (cm⁻¹): 3190, 1710, 1650
M S (C.I.): 349 m/e [M + H]⁺

### Analysis

| | | | | | | | |
|---|---|---|---|---|---|---|---|
| C₂₂H₂₄N₂O₂ | Found % | C | 75.20 | H | 6.75 | N | 7.85 |
| | Calc. % | C | 75.83 | H | 6.94 | N | 8.04 |

NMR δ(CDCl₃): 0.99 (3H, t N-CH₂CH₃); 1.3 - 2.9 (9H, m); 2.3 (2H, q N-CH₂CH₃); 5.13 (1H, s); 7.1 - 7.6 (6H, m); 7.9 (1H, m); 10.3 (1H, broad s)

In a similar way was also prepared:

### 6,11-dihydro-11-[(1-isopropyl-4-piperidinyl)-carbonyl]-5H-dibenz[b,e]-azepin-6-one

### (Compound 15)

M.p. 250°C
I R (cm⁻¹): 3180, 1710, 1650
M S (C.I.): 363 m/e [M + H]⁺

### Analysis

| | | | | | | | |
|---|---|---|---|---|---|---|---|
| C₂₃H₂₆N₂O₂ | Found % | C | 76.14 | H | 7.33 | N | 7.74 |
| | Calc. % | C | 76.21 | H | 7.23 | N | 7.73 |

NMR δ(CDCl₃): 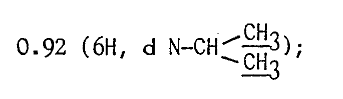 1.3 - 2.9 (10H, m); 5.1 (1H, s); 7.0 - 7.5 (6H, m); 7.9 (1H, m); 10.2 (1H, broad s).

The following not limitative examples of pharmaceutical compositions according to the invention are reported:

### Example 13

| Tablets | |
|---|---|
| - active ingredient | 25 mg |
| - lactose | 242 mg |
| - corn starch | 30 mg |
| - magnesium stearate | 3 mg |

Method of preparation: the active ingredient, lactose and corn starch were mixed and homogeneously moistened with water. After screening of the moist mass and drying in a tray drier, the mixture was again passed through a screen and magnesium stearate was added. Then the mixture was pressed into tablets weighing 300 mg each. Each tablet contains 25 mg of active ingredient.

### Example 14

| Capsules | |
|---|---|
| - active ingredient | 25 mg |
| - lactose | 173 mg |
| - magnesium stearate | 2 mg |

Method of preparation: the active ingredient was mixed with the auxiliary products, and the mixture was passed through a screen and mixed homogeneously in a suitable device. The resulting mixture was filled into hard gelatine capsules (200 mg per capsule); each capsule contains 25 mg of active ingredient.

### Example 15

| Ampoules | |
|---|---|
| - active ingredient | 1 mg |
| - sodium chloride | 8 mg |

Method of preparation: the active ingredient and sodium chloride were dissolved in an appropriate amount of water for injection. The resulting solution was filtered and filled into vials under sterile conditions.

### Example 16

| Suppositories | |
|---|---|
| - active ingredient | 50 mg |
| - semisynthetic glicerides of fatty acids | 1750 mg |

Method of preparation: the semisynthetic glicerides of fatty acids were melted and the active ingredient was added while stirring homogeneously. After cooling at a proper temperature the mass was poured into preformed moulds for suppositories weighing 1800 mg each. Each suppository contains 50 mg of active ingredient.

### Example 17

| Oral drops | |
|---|---|
| - active ingredient | 5 mg |
| - sorbitol | 350 mg |
| - propylene glycol | 200 mg |
| - citric acid | 1 mg |
| - sodium citrate | 3 mg |
| - demineralized water q.s. | 1 ml |

Method of preparation: the active ingredient, citric acid and sodium citrate were dissolved in a mixture of a proper amount of water and propylene glycol. Then sorbitol was added and the final solution ws filtered. The solution contains 1% of active ingredient and is administered by using a proper dropper.

## Claims

1. Compounds of general formula (I) 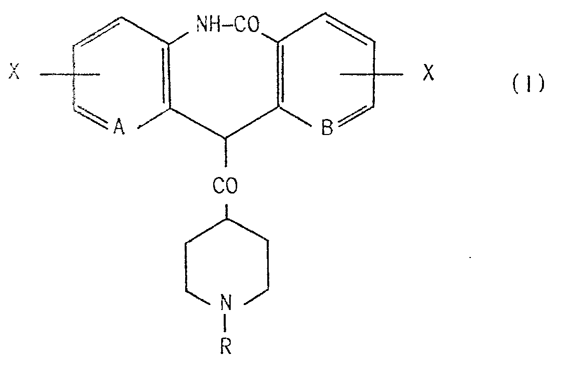 wherein
R represents a C₁₋₄ alkyl,
A and B represent independently nitrogen or carbon with the proviso that A and B cannot be nitrogen at the same time,
X represents a substituent at a carbon atom of the fused rings selected from hydrogen,halogen, methyl,
and acid addition salts thereof.

2. Compounds of general formula (I) according to claim 1, characterized in that R is methyl, X is hydrogen, chlorine or fluorine in position 1, 2, 9 and 10, A and B are independently nitrogen or carbon with the proviso that A and B cannot be nitrogen at the same time.

3. 6,11-Dihydro-11-[(1-methyl-4-piperidinyl)-carbonyl]-5H-dibenz[b,e]-azepin-6-one.

4. 1-Fluoro-6,11-dihydro-11-[(1-methyl-4-piperidinyl)-carbonyl]-5H-dibenz[b,e]-azepin-6-one.

5. 2-Chloro-6,11-dihydro-11-[(1-methyl-4-piperidinyl)-carbonyl]-5H-pyrido[2,3b]-benzazepin-6-one.

6. Physiologically compatible acid addition salts of compounds according to any of claims 1 to 5.

7. Salts according to claim 6, characterized in that the physiologically compatible acids are hydrochloric, fumaric or maleic acid.

8. Process for the preparation of compounds of general formula (I) according to claim 1, characterized in that a compound of general formula (II) 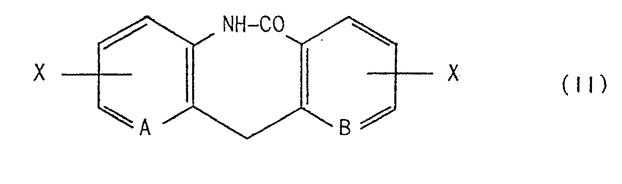 wherein A, B and X are as defined in claim 1, is at first converted "in situ" into the dilithium salt thereof in an inert organic solvant with organolithium compounds at temperatures of between -60°C and room temperature, and this lithium salt is subsequently reacted with a compound of general formula (III) 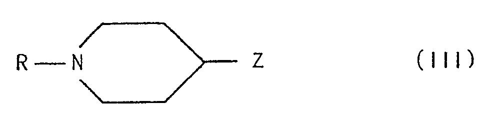 wherein R is as defined in claim 1 and Z represents a cyano or a CO-Y group, in which Y is chlorine, OR₄ or SR₄ in which R₄ is C₁₋₄ alkyl or phenyl.

9. Process according to claim 8 characterized in that the organolithium compounds are selected from lithium alkyl, lithium aryl or lithium dialkylamides.

10. Process according to claim 8 or 9, characterized in that the inert organic solvent is selected from tetrahydrofuran, diethyl ether, hexane and mixture of them.

11. Pharmaceutical compositions comprising as active ingredient at least one compound of general formula (I) of claim 1 or physiologically compatible acid addition salts thereof in association with pharmaceutical carriers or excipients.

12. Pharmaceutical compositions according to claim 11 as antiulcer - antisecretory agents.

13. Pharmaceutical compositions according to claim 11 for the use in the treatment of patients suffering from disorders of gastrointestinal tract.
